# EUROPEAN PATENT APPLICATION

(11) **EP 4 560 639 A1**
(43) Date of publication of application: **28.05.2025**
(21) Application number: 23843004.5
(22) Date of filing: 19.07.2023
(51) Int. Cl.: G16B 40/00

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 22.07.2022 JP 2022117573
(71) Applicant: FUJIFILM Corporation, Tokyo 106-8620 (JP)
(72) Inventor: TOMINAGA, Shunsuke, Ashigarakami-gun, Kanagawa 258-8577 (JP)
(74) Representative: HGF
(86) International application number: PCT/JP2023/026382
(87) International publication number: WO 2024/019081

(57) **Abstract**

An information processing device includes a processor configured to: compare multiple pieces of causal relationship information indicating a causal relationship of multiple events representing changes in a living body with an occurring event representing a change occurring in a subject which is a living body to which a pharmaceutical candidate substance was administered; extract corresponding causal relationship information containing the event corresponding to the occurring event from among the multiple pieces of causal relationship information; and carry out control to output the corresponding causal relationship information.

## Description

### BACKGROUND OF THE INVENTION

### 1. Field of the Invention

The technology of the present disclosure relates to an information processing device, an operating method for an information processing device, and an operating program for an information processing device.

### 2. Description of the Related Art

JP6407242B discloses a system that quantitatively calculates the magnitude of changes occurring in a living body caused by a pharmaceutical candidate substance (referred to as a "therapeutic compound" in JP6407242B) or the like, on the basis of on causal relationship information (referred to as a "network model" in JP6407242B) indicating a causal relationship of multiple events representing changes in the living body.

### SUMMARY OF THE INVENTION

In the related art, pharmaceutical candidate substances are evaluated as follows. First, a candidate substance is administered to a subject, such as a rat. A pathologist then identifies a change that occurs in the subject after administration of the candidate substance. Finally, the pathologist estimates the mechanism of action of a pharmaceutical manifestation or the mechanism of action of a toxic manifestation of the candidate substance, on the basis of on the identified change. Changes that occur in a subject after administration of a candidate substance are, for example, inflammation, canceration, or other morphological abnormalities that occur in the organs of the subject. The pathologist identifies morphological abnormalities by observing a specimen image showing a tissue specimen of an organ of the subject.

With recent advances in image analysis technology, automated identification of morphological abnormalities using specimen images by computer processing is getting closer to the practical stage. However, at present, estimation of the mechanism of action still relies entirely on the pathologist.

One embodiment according to the technology of the present disclosure provides an information processing device, an operating method for an information processing device, and an operating program for an information processing device that can lessen the burden on the pathologist and allow for efficient evaluation of pharmaceutical candidate substances.

An information processing device according to the present disclosure includes a processor configured to: compare multiple pieces of causal relationship information indicating a causal relationship of multiple events representing changes in a living body with an occurring event representing a change occurring in a subject which is a living body to which a pharmaceutical candidate substance was administered; extract corresponding causal relationship information containing the event corresponding to the occurring event from among the multiple pieces of causal relationship information; and carry out control to output the corresponding causal relationship information.

Preferably, the corresponding causal relationship information is information indicating a causal relationship of multiple events representing changes in a living body due to the toxicity of a pharmaceutical candidate substance in the past.

Preferably, the occurring event represents a morphological abnormality occurring in an organ of the subject after administration of the candidate substance.

Preferably, the processor is configured to identify the type of the morphological abnormality by performing image analysis on a specimen image showing a tissue specimen of an organ of the subject.

Preferably, the processor is configured to identify the types of multiple morphological abnormalities from a single specimen image.

Preferably, the processor is configured to use, in the image analysis, a machine learning model that accepts input of the specimen image and outputs in response an indication of whether the morphological abnormality is occurring.

Preferably, the processor is configured to, in a case where multiple pieces of the corresponding causal relationship information are extracted, derive a confidence level for each of the multiple pieces of the corresponding causal relationship information, and carry out control to output the corresponding causal relationship information on the basis of the confidence level.

Preferably, the confidence level is high to the extent that the corresponding causal relationship information contains a large number of events corresponding to the occurring event.

Preferably, the occurring event represents a morphological abnormality occurring in an organ of the subject after administration of the candidate substance, and the processor is configured to: identify the type of the morphological abnormality by using a machine learning model that accepts input of a specimen image showing a tissue specimen of an organ of the subject and outputs in response an indication of whether the morphological abnormality is occurring; and derive the confidence level on the basis of a predicted probability of whether the morphological abnormality is occurring according to the machine learning model.

Preferably, the processor is configured to, in a case where multiple pieces of the corresponding causal relationship information are extracted and a common event is present in at least two of the extracted multiple pieces of corresponding causal relationship information, carry out control to output the corresponding causal relationship information in which the common event is present, united at the common event.

Preferably, the causal relationship information is prepared for each multiple organs, and the processor is configured to carry out control to output the corresponding causal relationship information in which the common event is present, the corresponding causal relationship information being for different organs, united at the common event.

Preferably, the occurring event represents at least one from among a change in bodyweight of the subject, a change in food intake by the subject, and a change in the result of a clinical chemistry test on the subject.

Preferably, the causal relationship information contains an event involving a molecular change in the living body, and the processor is configured to distinguish an event involving a molecular change in the living body from another event when carrying out control to output the corresponding causal relationship information.

An operating method for an information processing device according to the present disclosure includes: comparing multiple pieces of causal relationship information indicating a causal relationship of multiple events representing changes in a living body with an occurring event representing a change occurring in a subject which is a living body to which a pharmaceutical candidate substance was administered; extracting corresponding causal relationship information containing the event corresponding to the occurring event from among the multiple pieces of causal relationship information; and carrying out control to output the corresponding causal relationship information.

An operating program for an information processing device according to the present disclosure causes a computer to execute a process including: comparing multiple pieces of causal relationship information indicating a causal relationship of multiple events representing changes in a living body with an occurring event representing a change occurring in a subject which is a living body to which a pharmaceutical candidate substance was administered; extracting corresponding causal relationship information containing the event corresponding to the occurring event from among the multiple pieces of causal relationship information; and carrying out control to output the corresponding causal relationship information. Advantageous Effects of Invention

According to the technology of the present disclosure, it is possible to provide an information processing device, an operating method for an information processing device, and an operating program for an information processing device that can lessen the burden on the pathologist and allow for efficient evaluation of pharmaceutical candidate substances.

### BRIEF DESCRIPTION OF THE DRAWINGS

Fig. 1 is a diagram illustrating an evaluation testing process, a specimen image, and an information processing device;
Fig. 2 is a block diagram illustrating a computer forming an information processing device;
Fig. 3 is a block diagram illustrating a processing unit of a CPU in an information processing device;
Fig. 4 is a diagram illustrating a morphological abnormality identification model;
Fig. 5 is a diagram illustrating patch images obtained by subdividing a target specimen image showing a liver specimen;
Fig. 6 is a diagram illustrating patch images obtained by subdividing a target specimen image showing a pancreas specimen;
Fig. 7 is a diagram illustrating a hyperplasia identification model;
Fig. 8 is a diagram illustrating how an aggregate identification result is generated by outputting identification results from identification models in response to the input of a patch image and aggregating the identification results;
Fig. 9 is a diagram illustrating how occurring event information is generated by further aggregating the aggregate identification results for patch images;
Fig. 10 is a diagram illustrating causal relationship information;
Fig. 11 is a diagram illustrating first liver causal relationship information;
Fig. 12 is a diagram illustrating first pancreas causal relationship information;
Fig. 13 is a diagram illustrating processing by a comparison and extraction unit;
Fig. 14 is a diagram illustrating processing by a comparison and extraction unit;
Fig. 15 is a diagram illustrating a target designation screen;
Fig. 16 is a diagram illustrating an analysis result display screen;
Fig. 17 is a flowchart illustrating a processing procedure by an information processing device;
Fig. 18 is a table illustrating the number of events corresponding to a morphological abnormality and the confidence level;
Fig. 19 is a diagram illustrating how multiple pieces of liver causal relationship information extracted as corresponding causal relationship information are displayed in descending order of confidence level;
Fig. 20 is a diagram illustrating how liver causal relationship information with a confidence level at or above a set value from among multiple pieces of liver causal relationship information extracted as corresponding causal relationship information is displayed in descending order of confidence level;
Fig. 21 is a table illustrating the mean predicted probability of a morphological abnormality corresponding to an event and the confidence level;
Fig. 22 is a diagram illustrating first liver causal relationship information and first pancreas causal relationship information in which a common event is present;
Fig. 23 is a diagram illustrating united causal relationship information in which the first liver causal relationship information and the first pancreas causal relationship information illustrated in Fig. 22 are united at the common event;
Fig. 24 is a diagram illustrating an analysis result display screen on which united causal relationship information is displayed;
Fig. 25 is a diagram illustrating occurring event information including changes in the bodyweight of the subject, changes in the food intake by the subject, and changes in the result of a clinical chemistry test on the subject; and
Fig. 26 is a diagram illustrating the handling of specimen images obtained by imaging slide specimens on which tissue specimens of multiple types of organs are placed.

### DESCRIPTION OF THE PREFERRED EMBODIMENTS

### Embodiment 1

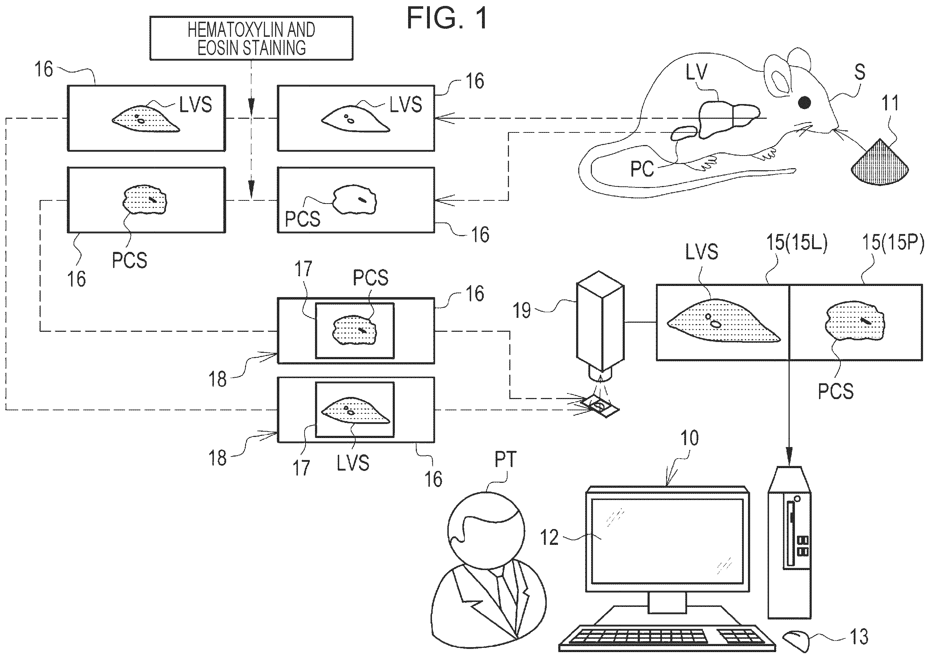

As illustrated by way of example in Fig. 1, an information processing device 10 according to the present disclosure is used to evaluate the efficacy and toxicity of a pharmaceutical candidate substance 11. The information processing device 10 is a desktop personal computer, for example, and is provided with a display 12 that displays various screens and an input device 13 such as a keyboard, a mouse, a touch panel, and/or a microphone for voice input. The information processing device 10 is installed in a pharmaceutical development facility, for example, and is operated by a pathologist PT involved in the development of pharmaceuticals at the pharmaceutical development facility.

A specimen image 15 is inputted into the information processing device 10. The specimen image 15 is an image for evaluating the efficacy and toxicity of a candidate substance 11. The specimen image 15 is generated by the following procedure, for example. First, a subject S such as a rat, which is a living body prepared for evaluation of the candidate substance 11, is autopsied, and tissue specimens of organs of the subject S, in this case a tissue specimen of a transverse section of the liver LV (hereinafter referred to as the liver specimen) LVS and a tissue sample of a transverse section of the pancreas PC (hereinafter referred to as the pancreas specimen) PCS, are collected. The collected liver specimen LVS and pancreas specimen PCS are then attached to microscope slides 16, after which the liver specimen LVS and the pancreas specimen PCS are stained, in this case with hematoxylin and eosin stain. Next, the stained liver specimen LVS and pancreas specimen PCS are covered by cover slips 17, thus completing slide specimens 18. The slide specimens 18 are placed in an imaging device 19 such as a digital/optical microscope, and the specimen image 15 is captured by the imaging device 19. The specimen image 15 obtained in this way is given subject identification data (ID) for uniquely identifying the subject S, a specimen image ID for uniquely identifying the specimen image 15, the imaging date and time, and the like.

In the following description, the specimen image 15 showing the liver specimen LVS is referred to as the specimen image 15L, and the specimen image 15 showing the pancreas specimen PCS is referred to as the specimen image 15P. The specimen images 15L and 15P are collectively referred to as the specimen image 15 when it is not particularly necessary to distinguish the images.

Fig. 1 is drawn as if one each of the liver specimen LVS and the pancreas specimen PCS is collected and the specimen images 15L and 15P are each captured only once, but in actuality, multiple transverse-section liver specimens LVS and pancreas specimens PCS are collected from a single subject S, and multiple specimen images 15L and 15P are also captured. Note that a tissue specimen is also referred to as a tissue section. Also, the staining may be staining by hematoxylin stain alone, staining by Nuclear Fast Red stain, or the like.

The following describes the administered group and the control group. The administered group includes multiple subjects S to which the candidate substance 11 was administered. The control group, contrary to the administered group, includes multiple subjects S to which the candidate substance 11 is not administered. In this example, to estimate the mechanism of action of a toxic manifestation of the candidate substance 11, images showing liver specimens LVS and pancreas specimens PCS of subjects S in the administered group are used as specimen images 15. The number of subjects S making up the administered group and the number of subjects S making up the control group are both around 5-10, for example. The subjects S making up the administered group and the subjects S making up the control group are subjects S with the same attributes and placed in the same rearing environment. The same attributes mean, for example, the same age in weeks and/or the same sex. The same attributes also include the same composition ratio of ages in weeks and/or the same composition ratio of sexes (such as five males and five females). The same rearing environment means, for example, the same food fed, the same temperature and humidity of the rearing space, and/or the same size of the rearing space. "The same" in the same rearing environment refers not only to being exactly the same but also to being the same in the sense of including error which is generally acceptable in the technical field to which the technology of the present disclosure belongs and which does not contradict the gist of the technology of the present disclosure.

Note that the administered group may include multiple groups with different administered amounts of the candidate substance 11. For example, the administered amounts of the candidate substance 11 are differentiated into three levels, such as a high administered amount group, a medium administered amount group, and a low administered amount group. This makes it possible to discern how the administered amount of the candidate substance 11 affects the subjects S.

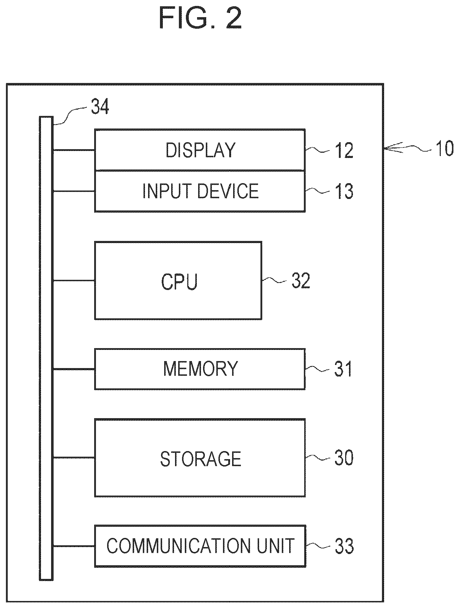

As illustrated by way of example in Fig. 2, a computer forming the information processing device 10 is provided with storage 30, memory 31, a central processing unit (CPU) 32, and a communication unit 33, in addition to the display 12 and the input device 13 described above. These components are interconnected via a bus line 34.

The storage 30 is a hard disk drive that is built into the computer forming the information processing device 10 or connected thereto via a cable or network. Alternatively, the storage 30 is a disk array combining multiple hard disk drives. In the storage 30, a control program such as an operating system, various application programs, various data associated with these programs, and the like are stored. Note that a solid-state drive may also be used instead of a hard disk drive.

The memory 31 is working memory used by the CPU 32 to execute processing. The CPU 32 loads a program stored in the storage 30 into the memory 31, and executes processing according to the program. This causes the CPU 32 to centrally control each component of the computer. The CPU 32 is an example of a "processor" according to the technology of the present disclosure. Note that the memory 31 may also be built into the CPU 32. The communication unit 33 controls the transfer of various information to and from external devices such as the imaging device 19.

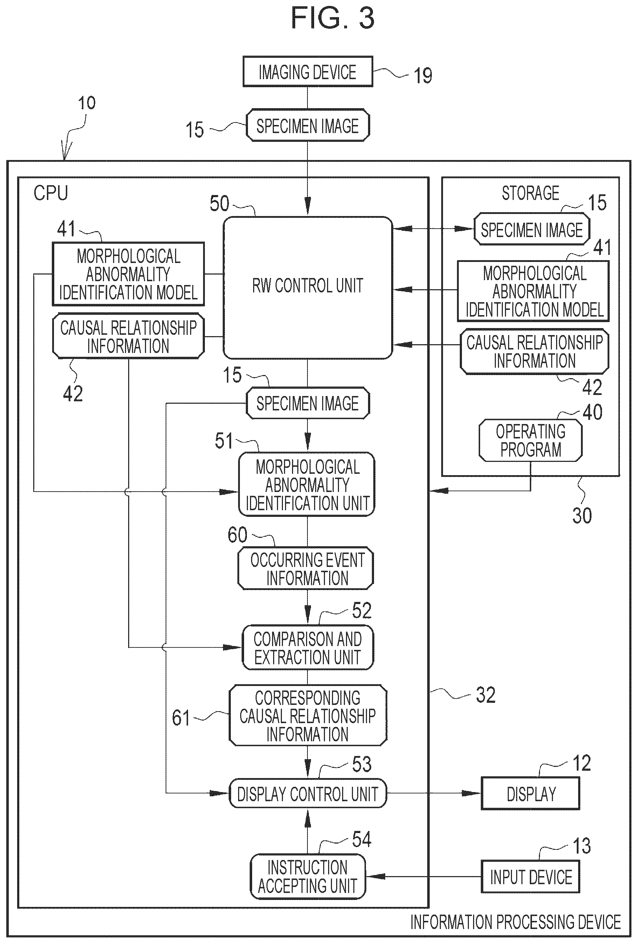

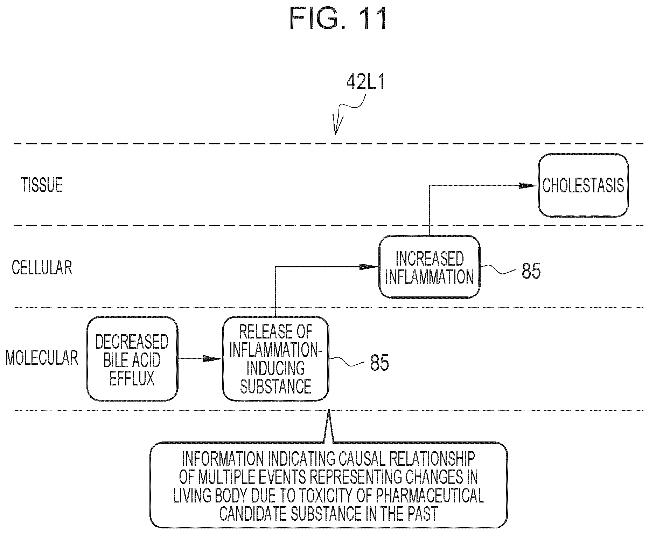

As illustrated by way of example in Fig. 3, an operating program 40 is stored in the storage 30 of the information processing device 10. The operating program 40 is an application program for causing the computer to function as the information processing device 10. That is, the operating program 40 is an example of an "operating program for an information processing device" according to the technology of the present disclosure. A morphological abnormality identification model 41, causal relationship information 42, and the like are also stored in the storage 30. The morphological abnormality identification model 41 is an example of a "machine learning model" according to the technology of the present disclosure. The causal relationship information 42 indicates a causal relationship of multiple events 85 representing changes in a living body (see Fig. 11 and elsewhere). "Living body" herein is a broad and general concept of a "living thing", not limited to the subject S. Accordingly, "living body" also includes animals of a different species from the subject S.

When the operating program 40 is launched, the CPU 32 of the computer forming the information processing device 10 cooperates with the memory 31 and the like to function as a read/write (hereinafter abbreviated to "RW") control unit 50, a morphological abnormality identification unit 51, a comparison and extraction unit 52, a display control unit 53, and an instruction accepting unit 54.

The RW control unit 50 controls the storing of various data to the storage 30 and the reading of various data in the storage 30. For example, the RW control unit 50 stores the specimen image 15 from the imaging device 19 in the storage 30. Note that since multiple specimen images 15 are obtained from a single subject S in actuality, multiple specimen images 15 with respect to a single subject S are stored in the storage 30.

The RW control unit 50 reads the specimen image 15 from the storage 30 according to a designation given by the pathologist PT via the input device 13. The RW control unit 50 outputs the read specimen image 15 to the morphological abnormality identification unit 51 and the display control unit 53. The specimen image 15 outputted to the morphological abnormality identification unit 51 and the like from the RW control unit 50 is subjected to, among other things, identification of types of morphological abnormalities occurring in the liver specimen LVS and pancreas specimen PCS. Hereinafter, the specimen image 15 subjected to, among other things, identification of types of morphological abnormalities occurring in the liver specimen LVS and pancreas specimen PCS is referred to as the target specimen image 15T.

The RW control unit 50 reads the morphological abnormality identification model 41 from the storage 30, and outputs the read morphological abnormality identification model 41 to the morphological abnormality identification unit 51. Also, the RW control unit 50 reads the causal relationship information 42 from the storage 30, and outputs the read causal relationship information 42 to the comparison and extraction unit 52.

The morphological abnormality identification unit 51 uses the morphological abnormality identification model 41 to identify types of morphological abnormalities occurring in the tissue specimen (liver specimen LVS and pancreas specimen PCS) shown in the target specimen image 15T. A morphological abnormality is a lesion not seen in normal tissue specimens, such as hyperplasia, infiltration, stasis, inflammation, a tumor, canceration, proliferation, hemorrhage, or glycogen depletion. The morphological abnormality identification unit 51 outputs occurring event information 60, including an identified type of morphological abnormality, to the comparison and extraction unit 52. An occurring event represents a change occurring in the subject S to which the candidate substance 11 was administered. In this example, an occurring event represents a morphological abnormality.

The comparison and extraction unit 52 compares the events 85 in the causal relationship information 42 from the RW control unit 50 with the morphological abnormality in the occurring event information 60. Causal relationship information 42 containing an event 85 corresponding to the morphological abnormality in the occurring event information 60 is then extracted as corresponding causal relationship information 61. The comparison and extraction unit 52 outputs the corresponding causal relationship information 61 to the display control unit 53.

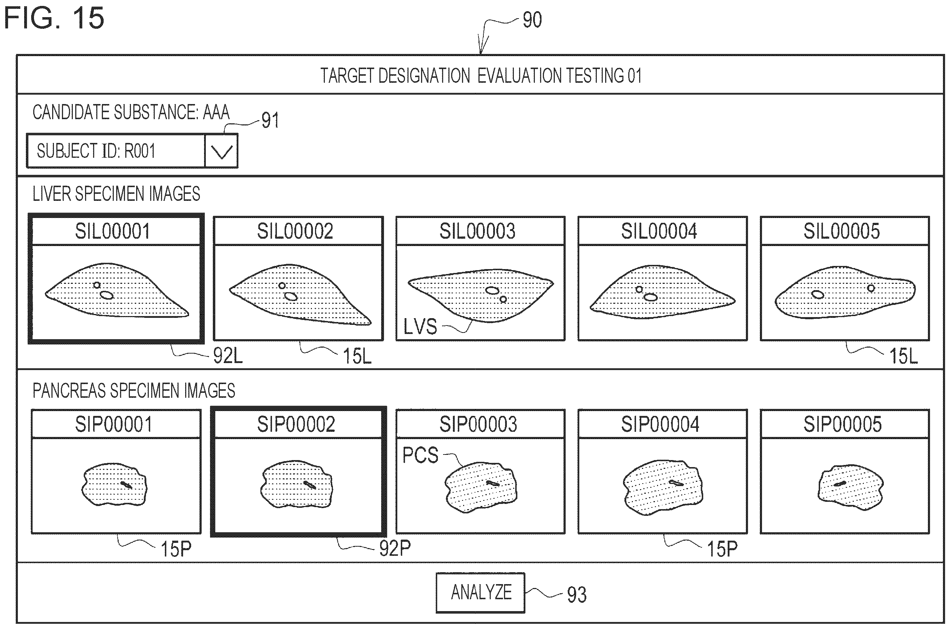

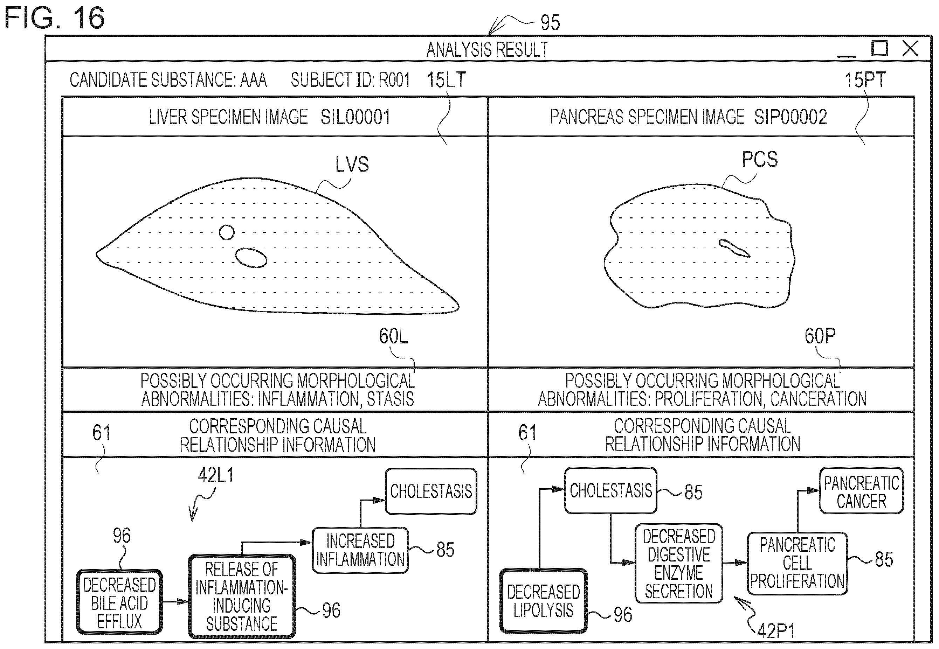

The display control unit 53 carries out control to display various screens on the display 12. The various screens include a target designation screen 90 for designating the target specimen image 15T (see Fig. 15), an analysis result display screen 95 on which the target specimen image 15T and the corresponding causal relationship information 61 are displayed (see Fig. 16), and the like. The instruction accepting unit 54 accepts various instructions given by the pathologist PT on the various screens via the input device 13.

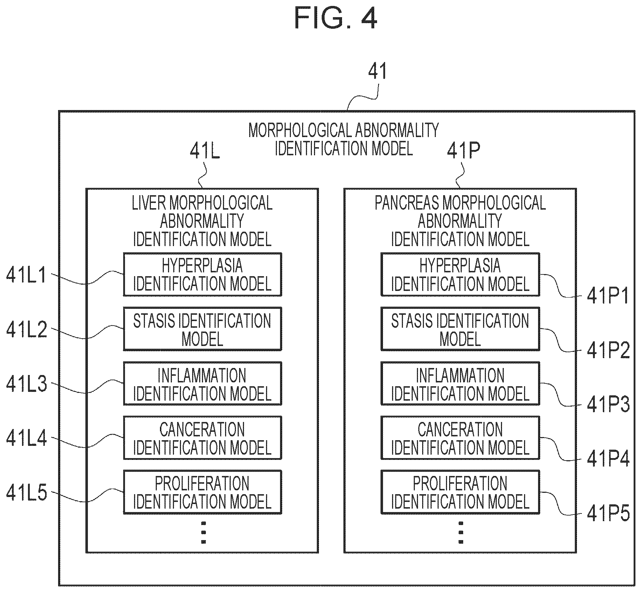

As illustrated by way of example in Fig. 4, the morphological abnormality identification model 41 includes a liver morphological abnormality identification model 41L and a pancreas morphological abnormality identification model 41P. The liver morphological abnormality identification model 41L and pancreas morphological abnormality identification model 41P further includes identification models for each of multiple morphological abnormalities. Namely, the liver morphological abnormality identification model 41L includes a hyperplasia identification model 41L1 for identifying hyperplasia, a stasis identification model 41L2 for identifying stasis, an inflammation identification model 41L3 for identifying inflammation, a canceration identification model 41L4 for identifying canceration, a proliferation identification model 41L5 for identifying proliferation, and the like. Similarly, the pancreas morphological abnormality identification model 41P includes a hyperplasia identification model 41P1 for identifying hyperplasia, a stasis identification model 41P2 for identifying stasis, an inflammation identification model 41P3 for identifying inflammation, a canceration identification model 41P4 for identifying canceration, a proliferation identification model 41P5 for identifying proliferation, and the like. Besides the above, the morphological abnormality identification model 41 may also include an infiltration identification model for identifying infiltration, a hemorrhage identification model for identifying hemorrhage, and the like.

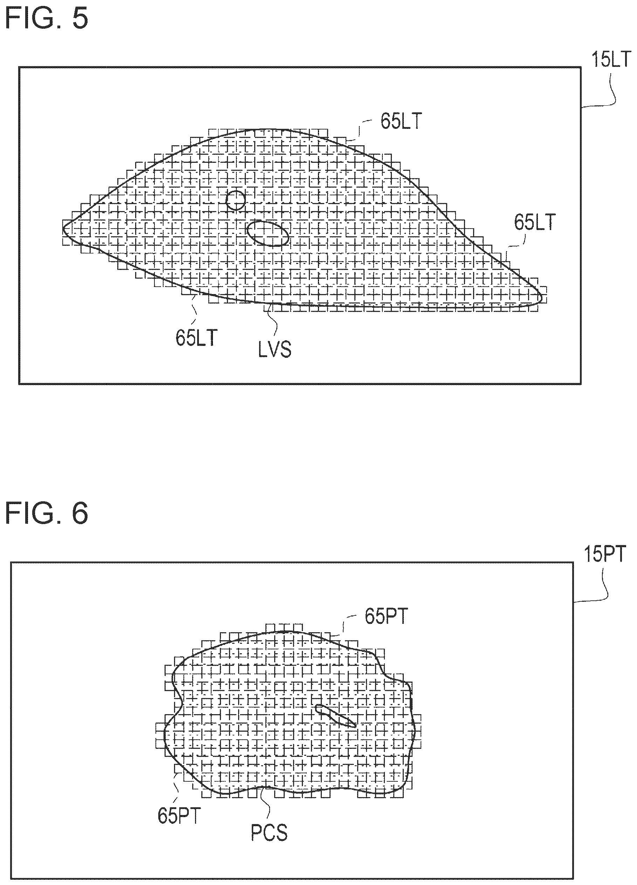

As illustrated by way of example in Fig. 5, the morphological abnormality identification unit 51 uses well-known image recognition technology to recognize the liver specimen LVS in the target specimen image 15T showing the liver specimen LVS (hereinafter referred to as the target specimen image 15LT), and subdivides the recognized liver specimen LVS into multiple patch images 65LT. Similarly, as illustrated by way of example in Fig. 6, the morphological abnormality identification unit 51 uses well-known image recognition technology to recognize the pancreas specimen PCS in the target specimen image 15T showing the pancreas specimen PCS (hereinafter referred to as the target specimen image 15PT), and subdivides the recognized pancreas specimen PCS into multiple patch images 65PT. The patch images 65LT and the patch images 65PT are of a preset size that can be handled by the liver morphological abnormality identification model 41L and the pancreas morphological abnormality identification model 41P. The morphological abnormality identification unit 51 assigns a patch image ID to each patch image 65LT and patch image 65PT. The morphological abnormality identification unit 51 also associates, with the patch image ID, information indicating the positions in the target specimen image 15LT or 15PT from which the patch images 65LT and 65PT were extracted, that is, position information about the patch images 65LT and 65PT. Note that in Figs. 5 and 6, the patch images 65LT and 65PT do not have regions that overlap with other patch images 65LT and 65PT, but the patch images 65LT and 65PT may also partially overlap with other patch images 65LT and 65PT.

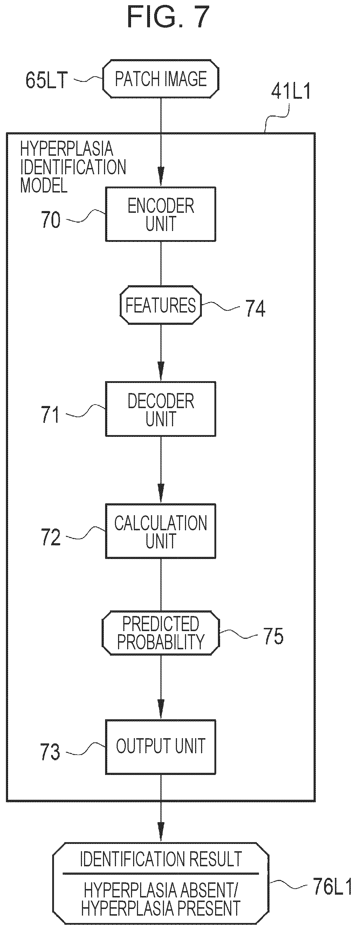

As illustrated by way of example in Fig. 7, the hyperplasia identification model 41L1 includes an encoder unit 70, a decoder unit 71, a calculation unit 72, and an output unit 73. The encoder unit 70 accepts the input of a patch image 65LT. The encoder unit 70 converts the patch image 65LT into features 74. The encoder unit 70 passes the features 74 to the decoder unit 71. The decoder unit 71 decodes the features 74.

As is well known, the encoder unit 70 has a convolutional layer that performs convolution processing using filters, a pooling layer that performs pooling processing such as max pooling, and the like. The decoder unit 71 is similar. That is, the hyperplasia identification model 41L1 is a convolutional neural network (CNN). The encoder unit 70 extracts the features 74 by repeatedly performing the convolution processing by the convolution layer and pooling processing by the pooling layer on the inputted patch image 65LT. The features 74 represent features of the shape and texture of the liver specimen LVS shown in the patch image 65LT. The features 74 are a set of multiple numerical values. That is, the features 74 are multidimensional data. The features 74 have a dimensionality of 512, 1024, or 2048, for example.

The calculation unit 72 calculates a predicted probability 75 of whether hyperplasia is occurring in the liver specimen LVS shown in the patch image 65LT, on the basis of data generated by the decoder unit 71 decoding the features 74. The predicted probability 75 is a numerical value between 0 and 1.0 (0% and 100%), for example, where a value closer to 1.0 indicates a higher probability that hyperplasia is occurring in the liver specimen LVS shown in the patch image 65LT. The calculation unit 72 outputs the predicted probability 75 to the output unit 73.

The output unit 73 outputs an identification result 76L1 according to the predicted probability 75. More specifically, the output unit 73 compares the predicted probability 75 to a preset threshold value. If the predicted probability 75 is less than the threshold value, the output unit 73 outputs an identification result 76L1 indicating that hyperplasia is not occurring in the liver specimen LVS shown in the patch image 65LT (illustrated as "hyperplasia absent" in Fig. 7). On the other hand, if the predicted probability 75 is equal to or greater than the threshold value, the output unit 73 outputs an identification result 76L1 indicating that hyperplasia is occurring in the liver specimen LVS shown in the patch image 65LT (illustrated as "hyperplasia present" in Fig. 7). The threshold value is 0.5, for example. Note that the other morphological abnormality identification models 41 such as the stasis identification model 41L2, the canceration identification model 41L4, and the hyperplasia identification model 41P1, and the inflammation identification model 41P3 have a configuration similar to the hyperplasia identification model 41L1, differing only in the content of the outputted identification result 76L. Accordingly, the hyperplasia identification model 41L1 is described as a representative example, and descriptions of the other morphological abnormality identification models 41 are omitted.

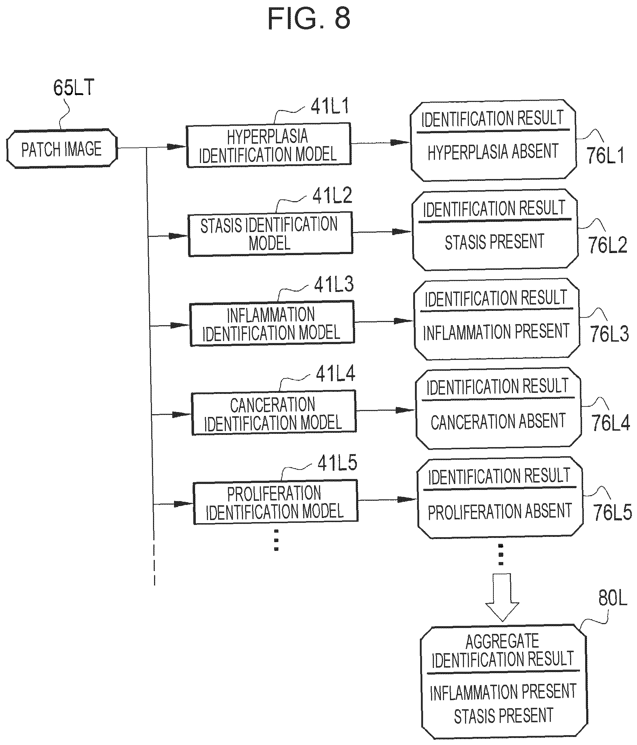

As illustrated by way of example in Fig. 8, the morphological abnormality identification unit 51 inputs a single patch image 65LT into all of the models making up the liver morphological abnormality identification model 41L (hyperplasia identification model 41L1, stasis identification model 41L2, inflammation identification model 41L3, canceration identification model 41L4, proliferation identification model 41L5, and the like). An identification result 76L is then outputted from each of the models. Specifically, an identification result 76L1 is outputted from the hyperplasia identification model 41L1, an identification result 76L2 is outputted from the stasis identification model 41L2, and an identification result 76L3 is outputted from the inflammation identification model 41L3. Also, an identification result 76L4 is outputted from the canceration identification model 41L4 and an identification result 76L5 is outputted from the proliferation identification model 41L5. The morphological abnormality identification unit 51 aggregates the multiple identification results 76L thus outputted from the models to generate an aggregate identification result 80L. The aggregate identification result 80L is the subset, from among the multiple identification results 76L outputted from the models, of identification results 76 containing an indication that a morphological abnormality is occurring in the liver specimen LVS shown in the patch image 65LT.

Fig. 8 illustrates an example in which the identification result 76L2 outputted from the stasis identification model 41L2 contains an indication of "stasis present" and the identification result 76L3 outputted from the inflammation identification model 41L3 contains an indication of "inflammation present", while the identification results 76L outputted from the other models all contain an indication of "morphological abnormality absent". In this case, the aggregate identification result 80L contains indications of "inflammation present" and "stasis present", as illustrated in the diagram. In this way, multiple types of morphological abnormalities may be identified from a single patch image 65LT in some cases.

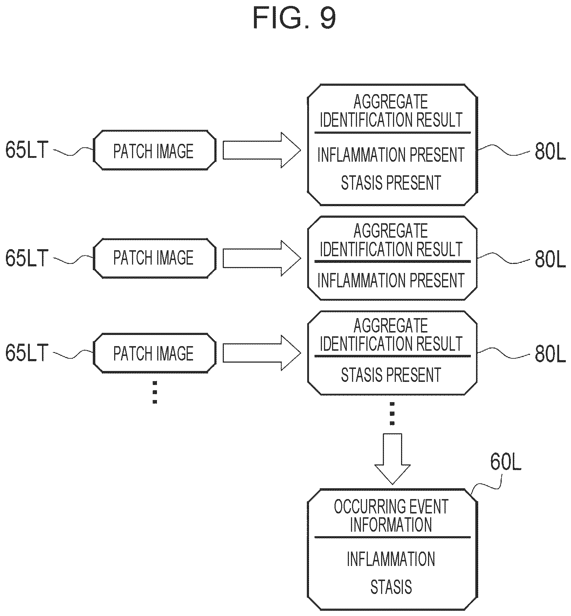

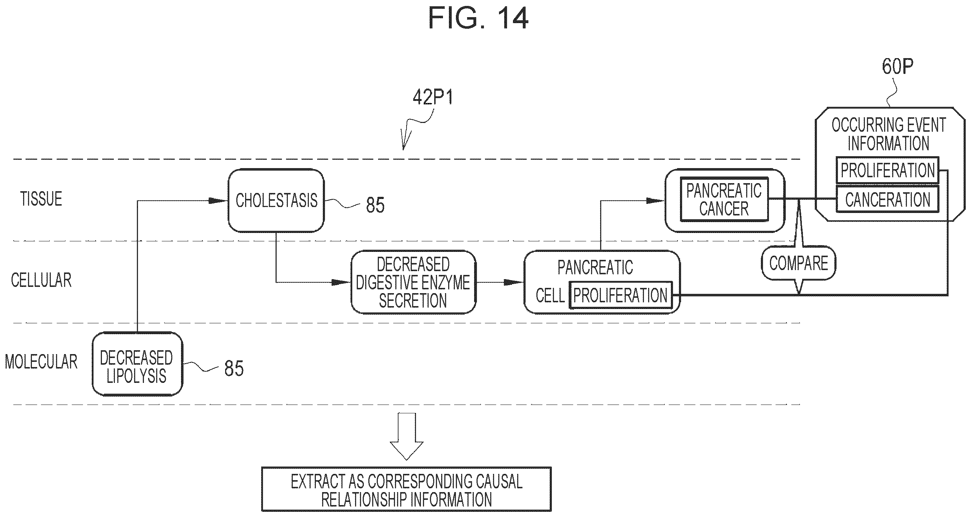

As illustrated by way of example in Fig. 9, the morphological abnormality identification unit 51 generates occurring event information 60L by further aggregating the aggregate identification results 80L of each of the patch images 65LT The occurring event information 60L is a summary of the morphological abnormalities deemed to be occurring in the aggregate identification results 80L. Fig. 9 illustrates an example of occurring event information 60L including the two morphological abnormalities of "inflammation" and "stasis" as occurring events. In this way, multiple types of morphological abnormalities may be identified from a single target specimen image 15LT in some cases. Although omitted from illustration in the drawings, the morphological abnormality identification unit 51 also processes the patch images 65PT in a manner similar to the patch images 65LT to generate occurring event information 60P (see Fig. 14).

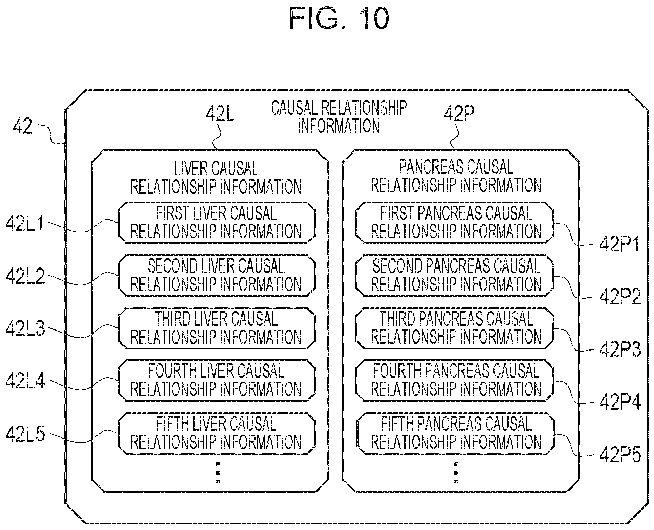

As illustrated by way of example in Fig. 10, the causal relationship information 42 includes liver causal relationship information 42L and pancreas causal relationship information 42P. The liver causal relationship information 42L includes first liver causal relationship information 42L1, second liver causal relationship information 42L2, third liver causal relationship information 42L3, fourth liver causal relationship information 42L4, fifth liver causal relationship information 42L5, and the like. The pancreas causal relationship information 42P similarly includes first pancreas causal relationship information 42P1, second pancreas causal relationship information 42P2, third pancreas causal relationship information 42P3, fourth pancreas causal relationship information 42P4, fifth pancreas causal relationship information 42P5, and the like. In this way, causal relationship information 42 is prepared for each of multiple organs.

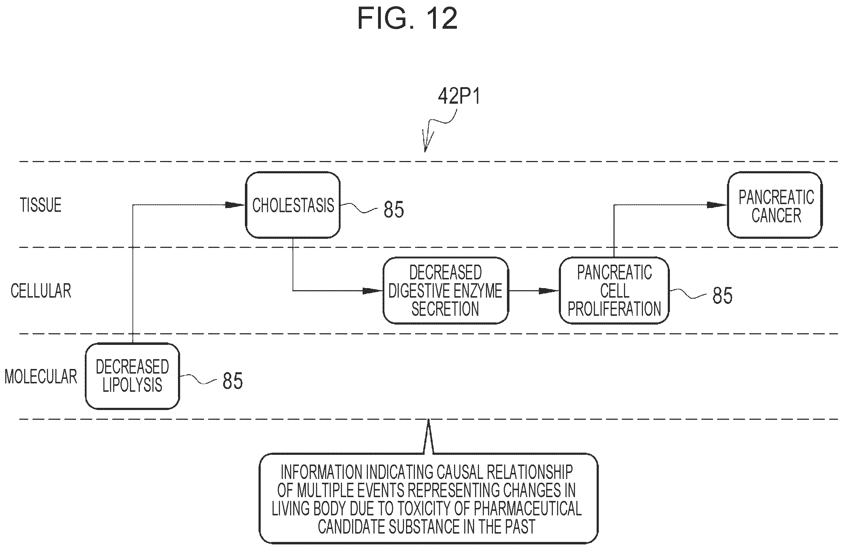

As illustrated by way of example in Figs. 11 and 12, the first liver causal relationship information 42L1 and first pancreas causal relationship information 42P1 contain multiple events 85 representing changes in the living body. The events 85 are connected to each other by arrows. The event 85 connected to the starting point an arrow indicates a cause, and the event 85 connected to the ending point of an arrow indicates an effect. Each 85 event is categorized as any of a change at the molecular, cellular, or tissue level of the living body.

In Fig. 11, the first liver causal relationship information 42L1 is information indicating a causal relationship of events 85 in which a "decreased bile acid efflux" event 85, which is a molecular-level change in the living body, is the beginning that connects to a "release of inflammation-inducing substance" event 85, which is also a molecular-level change, and an "increased inflammation" event 85, which is a cellular-level change, finally leading to a "cholestasis" event 85, which is a tissue-level change. That is, the first liver causal relationship information 42L1 is information indicating a causal relationship of multiple events 85 representing changes in a living body due to the toxicity of a pharmaceutical candidate substance in the past. Although omitted from illustration in the drawings, the other second liver causal relationship information 42L2, third liver causal relationship information 42L3, and the like are similar. Note that the "decreased bile acid efflux" event 85 and the "release of inflammation-inducing substance" event 85 are examples of an "event involving a molecular change in a living body" according to the technology of the present disclosure.

In Fig. 12, the first pancreas causal relationship information 42P1 is information indicating a causal relationship of events 85 in which a "decreased lipolysis" event 85, which is a molecular-level change in the living body, is the beginning that connects to a "cholestasis" event 85, which is a tissue-level change, a "decreased digestive enzyme secretion" event 85 and a "pancreatic cell proliferation" event 85, which are a cellular-level changes, finally leading to a "pancreatic cancer" event 85, which is a tissue-level change. That is, the first pancreas causal relationship information 42P1, like the first liver causal relationship information 42L1, is also information indicating a causal relationship of multiple events 85 representing changes in a living body due to the toxicity of a pharmaceutical candidate substance in the past. Although omitted from illustration in the drawings, the other second pancreas causal relationship information 42P2, third pancreas causal relationship information 42P3, and the like are similar. Note that the "decreased lipolysis" event 85 is an example of an "event involving a molecular change in a living body" according to the technology of the present disclosure.

The first pancreas causal relationship information 42P1 also has some events 85 related to the liver LV mixed in, as can be seen from the "cholestasis" event 85. However, since the final event 85 is "pancreatic cancer", which is an event 85 related to the pancreas PC, these events are classified as pancreas causal relationship information 42P.

Such causal relationship information 42 may be created by the pathologist PT on the basis of the results of evaluation testing of pharmaceutical candidate substances in the past. The causal relationship information 42 may also be obtained from a public database, such as the Adverse Outcome Pathway Wiki (AOP-Wiki), for example. Note that the causal relationship information 42 may also simply contain information about the events 85 only, and not indicate with arrows how the events 85 are related to each other as in the illustrated example.

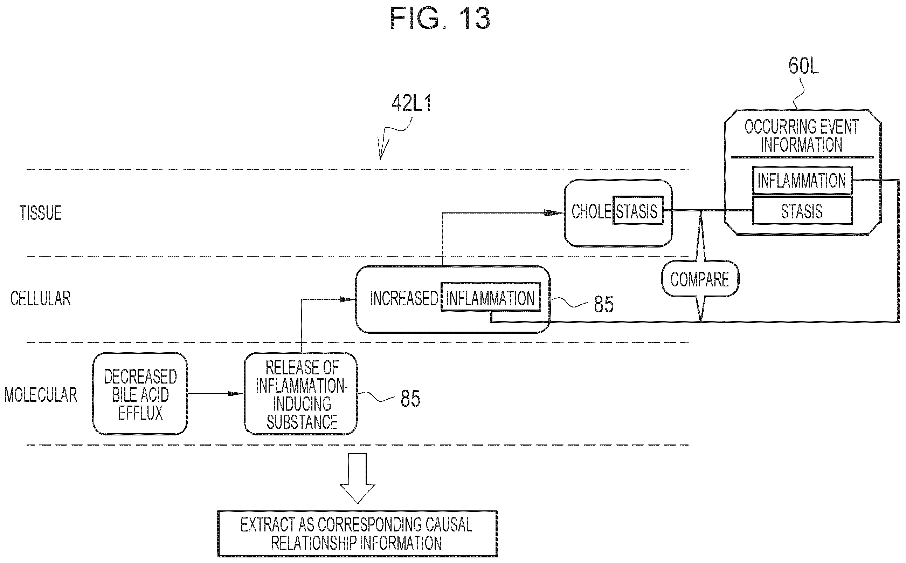

As illustrated by way of example in Figs. 13 and 14, the comparison and extraction unit 52 compares the causal relationship information 42 with the occurring event information 60. Fig. 13 illustrates how the first liver causal relationship information 42L1 and the occurring event information 60L are compared. Through the comparison, the comparison and extraction unit 52 concludes that the "increased inflammation" event 85 in the first liver causal relationship information 42L1 and the "inflammation" in the occurring event information 60L correspond to each other. Also, through the comparison, the comparison and extraction unit 52 concludes that the "cholestasis" event 85 in the first liver causal relationship information 42L1 and the "stasis" in the occurring event information 60L correspond to each other. In this case, since the first liver causal relationship information 42L1 contains events 85 that correspond to morphological abnormalities in the occurring event information 60L, the comparison and extraction unit 52 extracts the first liver causal relationship information 42L1 as corresponding causal relationship information 61. Note that, although omitted from illustration in the drawings, liver causal relationship information 42L not containing any events 85 that correspond to a morphological abnormality in the occurring event information 60L is obviously not extracted as corresponding causal relationship information 61 by the comparison and extraction unit 52.

Fig. 14 illustrates how the first pancreas causal relationship information 42P1 and the occurring event information 60P are compared. Through the comparison, the comparison and extraction unit 52 concludes that the "pancreatic cell proliferation" event 85 in the first pancreas causal relationship information 42P1 and the "proliferation" in the occurring event information 60P correspond to each other. Also, through the comparison, the comparison and extraction unit 52 concludes that the "pancreatic cancer" event 85 in the first pancreas causal relationship information 42P1 and the "canceration" in the occurring event information 60P correspond to each other. In this case, since the first pancreas causal relationship information 42P1 contains events 85 that correspond to morphological abnormalities in the occurring event information 60P, the comparison and extraction unit 52 extracts the first pancreas causal relationship information 42P1 as corresponding causal relationship information 61. Note that, although omitted from illustration in the drawings, pancreas causal relationship information 42P not containing any events 85 that correspond to a morphological abnormality in the occurring event information 60P is obviously not extracted as corresponding causal relationship information 61 by the comparison and extraction unit 52.

The display control unit 53 carries out control to display a target designation screen 90, an example of which is illustrated in Fig. 15, on the display 12. The display control unit 53 displays the target designation screen 90 when the pathologist PT gives a specimen image 15 display instruction via the input device 13, and this display instruction is accepted by the instruction accepting unit 54. On the target designation screen 90, multiple specimen images 15 are displayed side by side. These multiple specimen images 15 were obtained from a single subject S among the multiple subjects S making up the administered group. Fig. 15 illustrates an example in which five specimen images 15L with the specimen image IDs "SIL00001" to "SIL00005" and five specimen images 15P with the specimen image IDs "SIP00001" to "SIP00005" that were obtained from the subject S with the subject ID "R001" are displayed side by side. Note that the subject ID is a pull-down menu 91, allowing for switching of the subject S for whom specimen images 15 are to be displayed on the target designation screen 90.

The target designation screen 90 is a screen for designating one target specimen image 15LT from among the multiple specimen images 15L. The target designation screen 90 is also a screen for designating one target specimen image 15PT from among the multiple specimen images 15P. The target designation screen 90 is provided with one selection frame 92L that can be moved among the specimen images 15L, and one selection frame 92P that can be moved among the specimen images 15P. Also, an analyze button 93 is provided at the bottom of the target designation screen 90. The pathologist PT selects the analyze button 93 after aligning the selection frames 92L and 92P with the desired specimen images 15L and 15P. This causes the morphological abnormality identification unit 51 to identify the type of morphological abnormality, causes the comparison and extraction unit 52 to extract corresponding causal relationship information 61, and the like using the specimen images 15L and 15P with which the selection frames 92L and 92P are aligned as the target specimen images 15LT and 15PT.

When the corresponding causal relationship information 61 is inputted from the comparison and extraction unit 52, the display control unit 53 carries out control to display an analysis result display screen 95, an example of which is illustrated in Fig. 16, on the display 12. On the analysis result display screen 95, the target specimen images 15LT and 15PT, the occurring event information 60L and 60P (illustrated as "possibly occurring morphological abnormalities" in Fig. 16), and the corresponding causal relationship information 61 are displayed. Fig. 16 illustrates an example in which the first liver causal relationship information 42L1 illustrated in Figs. 11 and 13 and the first pancreas causal relationship information 42P1 illustrated in Figs. 12 and 14 are displayed as the corresponding causal relationship information 61. The control to display the analysis result display screen 95 including the corresponding causal relationship information 61 on the display 12 is an example of "control to output corresponding causal relationship information" according to the technology of the present disclosure.

The display control unit 53 displays an emphasis frame 96 around events 85 involving a molecular change in the living body to distinguish these events 85 from other events 85 among the events 85 in the corresponding causal relationship information 61. In Fig. 16, the events 85 involving a molecular change in the living body are the "decreased bile acid efflux" and "release of inflammation-inducing substance" events 85 in the first liver causal relationship information 42L1 and the "decreased lipolysis" event 85 in the first pancreas causal relationship information 42P1. Note that the adopted method of distinguishing events 85 involving a molecular change in the living body from other events 85 may also be a method such as changing the color of events 85 involving a molecular change in the living body to a different color than other events 85, making events 85 involving a molecular change in the living body larger in size than other events 85, or causing events 85 involving a molecular change in the living body to blink.

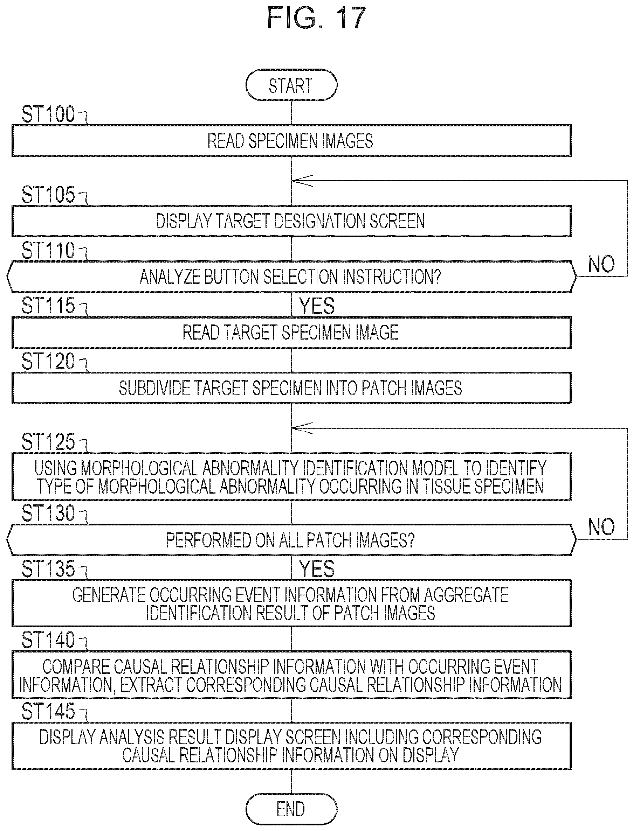

Next, the action according to the above configuration will be described with reference to the flowchart illustrated by way of example in Fig. 17. First, when the operating program 40 is launched in the information processing device 10, the CPU 32 of the information processing device 10 functions as the RW control unit 50, the morphological abnormality identification unit 51, the comparison and extraction unit 52, the display control unit 53, and the instruction accepting unit 54, as illustrated in Fig. 3.

The specimen image 15L showing the liver specimen LVS of the subject S and the specimen image 15P showing the pancreas specimen PCS of the subject S are captured by the imaging device 19. The specimen images 15L and 15P are outputted from the imaging device 19 to the information processing device 10. In the information processing device 10, the specimen images 15L and 15P from the imaging device 19 are stored in the storage 30 by the RW control unit 50.

When the pathologist PT gives a specimen image 15 display instruction via the input device 13, the specimen images 15 designated by the display instruction are read from the storage 30 by the RW control unit 50 (step ST100). The specimen images 15 are outputted from the RW control unit 50 to the display control unit 53. As illustrated in Fig. 15, the specimen images 15 are displayed on the display 12 via the target designation screen 90, under control by the display control unit 53 (step ST105).

When the pathologist PT aligns the selection frames 92L and the 92P with the desired specimen images 15 on the target designation screen 90 and selects the analyze button 93 (step ST110, YES), the specimen images 15L and 15P with which the selection frames 92L and 92P are currently aligned are read from the storage 30 by the RW control unit 50 as the target specimen images 15LT and 15PT (step ST115). The target specimen images 15LT and 15PT are outputted from the RW control unit 50 to the morphological abnormality identification unit 51 and the display control unit 53.

The morphological abnormality identification model 41 is read from the storage 30 by the RW control unit 50, and the read morphological abnormality identification model 41 is outputted to the morphological abnormality identification unit 51. Also, the causal relationship information 42 is read from the storage 30 by the RW control unit 50, and the read causal relationship information 42 is outputted to the comparison and extraction unit 52.

As illustrated in Figs. 5 and 6, in the morphological abnormality identification unit 51, the target specimen images 15LT and 15PT are subdivided into multiple patch images 65LT and 65PT (step ST120). Next, as illustrated in Fig. 8, in the morphological abnormality identification unit 51, a patch image 65LT is inputted into the hyperplasia identification model 41L1 and the like, and an identification result 76L1 and the like is outputted from the hyperplasia identification model 41L1 and the like. Then, in the morphological abnormality identification unit 51, the multiple identification results 76L outputted from the models are aggregated, and an aggregate identification result 80L is generated. Thus, the type of morphological abnormality occurring in the liver specimen LVS shown in the patch image 65LT is identified (step ST125). The processing in step ST125 is performed with respect to all of the patch images 65LT. Similar processing is also performed with respect to the patch images 65PT.

After the processing in step ST125 is performed with respect to all of the patch images 65LT and 65PT (step ST130, YES), as illustrated in Fig. 9, in the morphological abnormality identification unit 51, occurring event information 60L is generated from the aggregate identification result 80L of the patch images 65LT (step ST135). Similar processing is also performed with respect to the patch images 65PT, and occurring event information 60P is generated (step ST135). The occurring event information 60L and 60P are outputted from the morphological abnormality identification unit 51 to the comparison and extraction unit 52.

In the comparison and extraction unit 52, as illustrated in Figs. 13 and 14, the causal relationship information 42 and the occurring event information 60 are compared, and corresponding causal relationship information 61 is extracted from among multiple pieces of causal relationship information 42 (step ST140). The corresponding causal relationship information 61 is outputted from the comparison and extraction unit 52 to the display control unit 53.

Under control by the display control unit 53, the analysis result display screen 95 illustrated in Fig. 16 is displayed on the display 12 (step ST145). The corresponding causal relationship information 61 is included on the analysis result display screen 95. The pathologist PT views the analysis result display screen 95 and refers to the corresponding causal relationship information 61 and the like to estimate the mechanism of action of a toxic manifestation of the candidate substance 11.

As described above, the CPU 32 of the information processing device 10 is provided with the comparison and extraction unit 52 and the display control unit 53. The comparison and extraction unit 52 compares multiple pieces of causal relationship information 42 indicating a causal relationship of multiple events 85 representing changes in a living body with an occurring event representing a change occurring in a subject S which is a living body to which the pharmaceutical candidate substance 11 was administered. With this arrangement, the comparison and extraction unit 52 extracts corresponding causal relationship information 61 containing an event 85 corresponding to a morphological abnormality from among the multiple pieces of causal relationship information 42. The display control unit 53 carries out control to display the analysis result display screen 95 including the corresponding causal relationship information 61 on the display 12. The corresponding causal relationship information 61 greatly helps the pathologist PT estimate the mechanism of action of a toxic manifestation of the candidate substance 11. Accordingly, the burden on the pathologist PT is lessened, allowing for efficient evaluation of the candidate substance 11.

As illustrated in Figs. 11 and 12, the causal relationship information 42 is information indicating a causal relationship of multiple events 85 representing changes in a living body due to the toxicity of a pharmaceutical candidate substance in the past. This allows the pathologist PT to make progress in estimating the mechanism of action of a toxic manifestation of the candidate substance 11. Note that causal relationship information 42 indicating a causal relationship of multiple events 85 representing changes in a living body due to the efficacy of a pharmaceutical candidate substance in the past may also be used instead of, or in addition to, the causal relationship information 42 indicating a causal relationship of multiple events 85 representing changes in a living body due to the toxicity of a pharmaceutical candidate substance in the past.

The occurring events represent morphological abnormalities occurring in organs, in this case the liver specimen LVS and the pancreas specimen PCS, of the subject S after administration of the candidate substance 11. Relatively many of the events 85 in the causal relationship information 42 are related to morphological abnormalities, such as "increased inflammation" and "cholestasis" in the first liver causal relationship information 42L1 or "pancreatic cell proliferation" and "pancreatic cancer" in the first pancreas causal relationship information 42P1 of the illustrated example. Therefore, by configuring the occurring events to be morphological abnormalities, more plausible corresponding causal relationship information 61 can be extracted, which allows the pathologist PT to make further progress in estimating the mechanism of action of a toxic manifestation of the candidate substance 11.

The morphological abnormality identification unit 51 identifies the type of morphological abnormality by performing image analysis on the target specimen images 15LT and 15PT showing tissue specimens of organs, in this case the liver specimen LVS and the pancreas specimen PCS, of the subject S. Therefore, the burden on the pathologist PT can be lessened further, as compared to the case where the pathologist PT identifies the type of morphological abnormality by observing the target specimen images 15LT and 15PT.

The morphological abnormality identification unit 51 identifies the types of multiple morphological abnormalities with respect to a single target specimen image 15LT or 15PT. Therefore, corresponding causal relationship information 61 that is more helpful for estimating the mechanism of action of a toxic manifestation of the candidate substance 11 can be extracted, as compared to the case where always only one type of morphological abnormality can be identified with respect to a single target specimen image 15LT or 15PT.

In the image analysis, the morphological abnormality identification unit 51 uses the morphological abnormality identification model 41 that outputs an indication of whether a morphological abnormality is occurring according to the input of the target specimen images 15LT and 15PT (patch images 65LT and 65PT). Recently, machine learning models such as the morphological abnormality identification model 41 can be prepared easily with relatively high prediction accuracy. Therefore, types of morphological abnormalities can be identified easily and accurately. Note that by using a well-known technology such as pattern recognition technology, types of morphological abnormalities may also be estimated without using a machine learning model.

The causal relationship information 42 contains events 85 involving molecular changes in a living body. As illustrated in Fig. 16, when carrying out control to display the corresponding causal relationship information 61, the display control unit 53 displays an emphasis frame 96 around events 85 involving a molecular change in the living body to distinguish these events 85 from other events 85. The events 85 involving a molecular change in the living body are considered to be promising candidates for pharmacodynamic biomarkers, which are important for estimating the mechanism of action of a toxic manifestation of the candidate substance 11. Therefore, by distinguishing events 85 involving a molecular change in the living body from other events 85, the attention of the pathologist PT can be directed to the events 85 involving a molecular change in the living body

### Embodiment 2_1

Embodiment 1 above illustrates an example in which one piece of corresponding causal relationship information 61 is extracted for each of the liver LV and the pancreas PC, but the configuration is not limited thereto. The comparison and extraction unit 52 may also extract multiple pieces of corresponding causal relationship information 61.

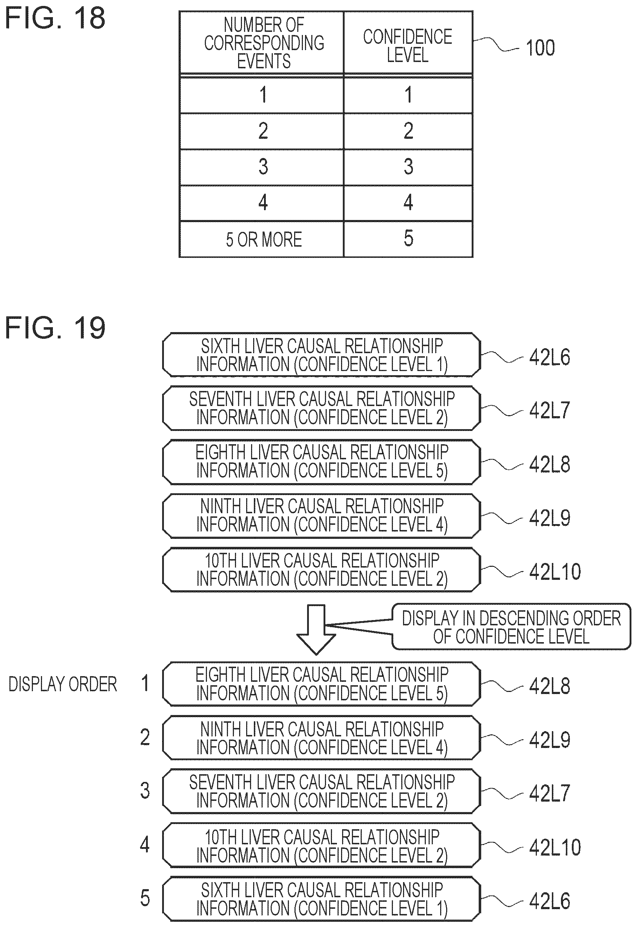

In the case where the comparison and extraction unit 52 extracts multiple pieces of corresponding causal relationship information 61, the display control unit 53 derives a confidence level for each of the multiple pieces of corresponding causal relationship information 61 on the basis of a table 100 illustrated by way of example in Fig. 18. The table 100 is data in which a confidence level is registered according to the number of events 85 corresponding to a morphological abnormality in the occurring event information 60 (hereinafter referred to as the number of corresponding events). For example, the confidence level is 1 when the number of corresponding events is 1, the confidence level is 4 when the number of corresponding events is 4, and the confidence level is 5 when the number of corresponding events is 5 or more. The higher the value of the confidence level for the corresponding causal relationship information 61 is, the higher the confidence level for the information is. To give an example in terms of the first liver causal relationship information 42L1 illustrated in Fig. 13 and the like and the first pancreas causal relationship information 42P1 illustrated in Fig. 14 and the like, in each of the above information, the number of corresponding events is 2, and thus the confidence level is 2.

As illustrated by way of example in Fig. 19, in the case where the comparison and extraction unit 52 extracts multiple pieces of corresponding causal relationship information 61, the display control unit 53 displays the corresponding causal relationship information 61 in descending order of confidence level on the analysis result display screen 95. Fig. 19 illustrates an example in which five pieces of liver causal relationship information 42L, namely sixth liver causal relationship information 42L6, seventh liver causal relationship information 42L7, eighth liver causal relationship information 42L8, ninth liver causal relationship information 42L9, and 10th liver causal relationship information 42L10, are extracted as the corresponding causal relationship information 61. Also, Fig. 19 illustrates an example in which the sixth liver causal relationship information 42L6 has a confidence level of 1, the seventh liver causal relationship information 42L7 and the 10th liver causal relationship information 42L10 each have a confidence level of 2, the eighth liver causal relationship information 42L8 has a confidence level of 5, and the ninth liver causal relationship information 42L9 has a confidence level of 4.

In this case, the display control unit 53 puts the eighth liver causal relationship information 42L8 with the highest confidence level of 5 in first place in the display order on the analysis result display screen 95. Thereafter, the ninth liver causal relationship information 42L9 is put in second place in the display order, the seventh liver causal relationship information 42L7 is put in third place in the display order, the 10th liver causal relationship information 42L10 is put in fourth place in the display order, and finally, the sixth liver causal relationship information 42L6 with the lowest confidence level of 1 is put in fifth place in the display order.

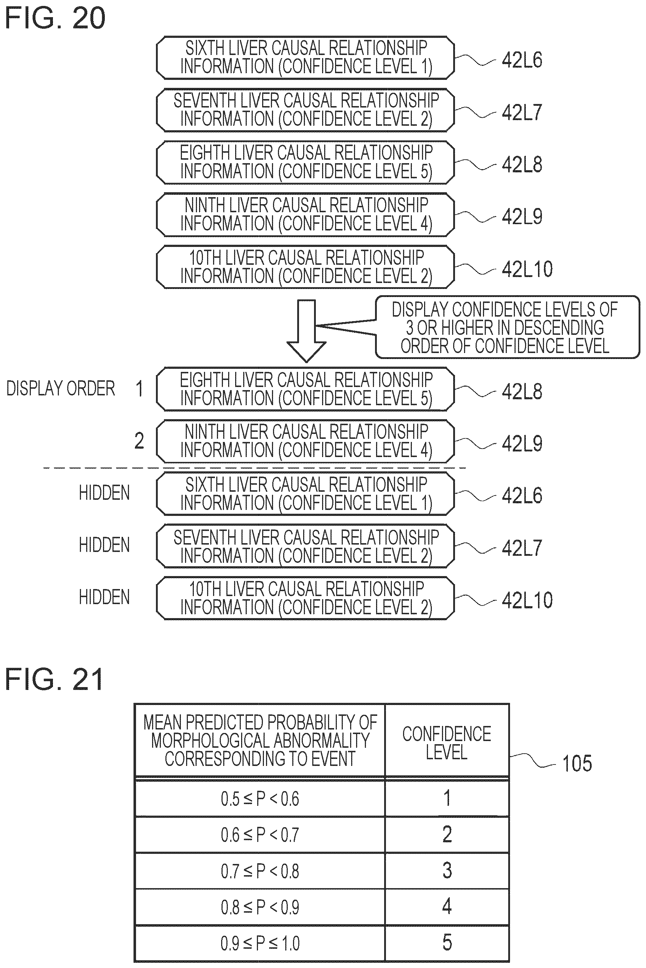

Alternatively, as illustrated by way of example in Fig. 20, in the case where the comparison and extraction unit 52 extracts multiple pieces of corresponding causal relationship information 61, the display control unit 53 displays the corresponding causal relationship information 61 for which the confidence level is at least a set value, in this case 3 or higher, in descending order of confidence level on the analysis result display screen 95. In Fig. 20, the extracted corresponding causal relationship information 61 and confidence levels are the same as those of the example in Fig. 19. In this case, the display control unit 53 puts the eighth liver causal relationship information 42L8 with the highest confidence level of 5 in first place in the display order on the analysis result display screen 95, and puts the ninth liver causal relationship information 42L9 with the next-highest confidence level of 4 in second place in the display order. The sixth liver causal relationship information 42L6, seventh liver causal relationship information 42L7, and 10th liver causal relationship information 42L10 for which the confidence level is 1 or 2, which is less than the set value of 3, are hidden.

In this way, in embodiment 2_1, in the case where the comparison and extraction unit 52 extracts multiple pieces of corresponding causal relationship information 61, the display control unit 53 derives a confidence level for each of the multiple pieces of corresponding causal relationship information 61. Control is then carried out to display the corresponding causal relationship information 61 on the basis of the confidence levels. Therefore, multiple pieces of corresponding causal relationship information 61 can be displayed in descending order of confidence level, as illustrated in Fig. 19, and/or corresponding causal relationship information 61 for which the confidence level is less than a set value can be hidden, as illustrated in Fig. 20. This allows the pathologist PT to make further progress in estimating the mechanism of action of a toxic manifestation of the candidate substance 11.

The confidence level is high to the extent that the corresponding causal relationship information 61 contains a large number of corresponding events. Therefore, corresponding causal relationship information 61 with a large number of corresponding events can be displayed with priority over other corresponding causal relationship information 61. Corresponding causal relationship information 61 with a large number of corresponding events is thought to be more helpful for estimating the mechanism of action of a toxic manifestation of the candidate substance 11. Accordingly, this allows the pathologist PT to make further progress in estimating the mechanism of action of a toxic manifestation of the candidate substance 11. Note that the confidence level according to the number of corresponding events may also be displayed on the analysis result display screen 95.

### Embodiment 2_2

In the present embodiment, in the case where the comparison and extraction unit 52 extracts multiple pieces of corresponding causal relationship information 61, the display control unit 53 derives a confidence level for each of the multiple pieces of corresponding causal relationship information 61 on the basis of a table 105 illustrated by way of example in Fig. 21. The table 105 is data in which a confidence level is registered according to the mean predicted probability 75 of a morphological abnormality corresponding to an event 85 in the corresponding causal relationship information 61. For example, the confidence level is 1 when the mean predicted probability 75 is equal to or greater than 0.5 and less than 0.6, the confidence level is 3 when the mean predicted probability 75 is equal to or greater than 0.7 and less than 0.8, and the confidence level is 5 when the mean predicted probability 75 is equal to or greater than 0.9 and less than or equal to 1.0. In this case, in the same manner as in embodiment 2_1 above, the higher the value of the confidence level for the corresponding causal relationship information 61 is, the higher the confidence level for the information is.

The mean of the predicted probability 75 is calculated as follows. The first liver causal relationship information 42L1 illustrated in Fig. 13 and the like will be used again as an example. Consider the case in which "inflammation", which is the morphological abnormality corresponding to the "increased inflammation" event 85, has a predicted probability 75 of, for example, 0.85 according to the inflammation identification model 41L3, and "stasis", which is the morphological abnormality corresponding to the "cholestasis" event 85, has a predicted probability 75 of, for example, 0.65 according to the stasis identification model 41L2. In this case, the mean of the predicted probability 75 is (0.85 + 0.65) / 2 = 0.75. Note that the predicted probability 75 of "inflammation" according to the inflammation identification model 41L3 is a representative value of the predicted probabilities 75 obtained from all of the patch images 65LT for which the identification result 76L3 containing an indication of "inflammation present" was outputted. Similarly, the predicted probability 75 of "stasis" according to the stasis identification model 41L2 is a representative value of the predicted probabilities 75 obtained from all of the patch images 65LT for which the identification result 76L2 containing an indication of "stasis present" was outputted. The representative value is the mean, maximum, median, minimum, and the like.

In the present embodiment, like the case of embodiment 2_1, the display control unit 53 displays multiple pieces of corresponding causal relationship information 61 in descending order of confidence level and/or hides corresponding causal relationship information 61 for which the confidence level is less than a set value. Like the case of embodiment 2_1, this makes it possible to obtain the effect whereby the pathologist PT makes further progress in estimating the mechanism of action of a toxic manifestation of the candidate substance 11.

In this way, in embodiment 2_2, the display control unit 53 derives the confidence level on the basis of the predicted probability 75 of whether a morphological abnormality is occurring according to the morphological abnormality identification model 41. Therefore, corresponding causal relationship information 61 with a high mean predicted probability 75 can be displayed with priority over other corresponding causal relationship information 61. Corresponding causal relationship information 61 with a high mean predicted probability 75 is thought to be more helpful for estimating the mechanism of action of a toxic manifestation of the candidate substance 11. Accordingly, this allows the pathologist PT to make further progress in estimating the mechanism of action of a toxic manifestation of the candidate substance 11. Note that the confidence level according to the mean predicted probability 75 or the mean predicted probability 75 itself may also be displayed on the analysis result display screen 95. Also, the maximum, median, minimum, and the like may be used instead of the mean predicted probability 75 of a morphological abnormality corresponding to events 85 in the corresponding causal relationship information 61.

Embodiment 2_1 and embodiment 2_2 may also be carried out in a combined manner. In this case, the display control unit 53 derives the confidence level on the basis of data in which a confidence level is registered according to the number of corresponding events and the mean predicted probability 75. Alternatively, the display control unit 53 may derive the confidence level by solving a formula that takes the number of corresponding events and the mean predicted probability 75 as parameters.

The confidence level may also be derived on the basis of the ratio of the number of corresponding events and the total number of events 85 making up the corresponding causal relationship information 61. In this case, for example, Fisher's exact test is performed, and the p-value calculated thereby is subtracted from 1 to obtain the confidence level.

### Embodiment 3

In the present embodiment, in the case where the comparison and extraction unit 52 extracts multiple pieces of corresponding causal relationship information 61 and a common event 85 is present in at least two of the extracted multiple pieces of corresponding causal relationship information 61, the display control unit 53 unites the corresponding causal relationship information 61 in which the common event 85 is present, at the common event 85.

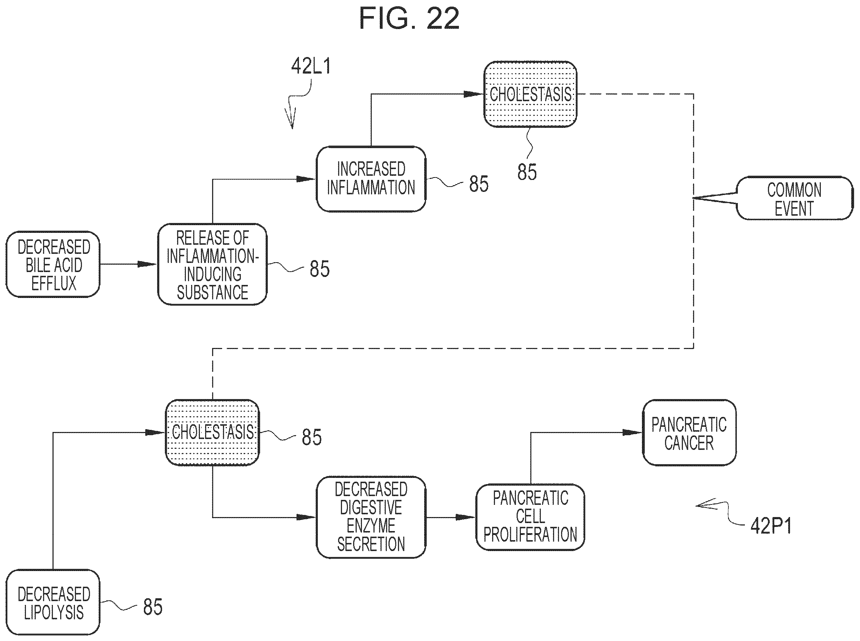

Fig. 22 illustrates an example in which, as in embodiment 1 above, the comparison and extraction unit 52 has extracted the first liver causal relationship information 42L1 and the first pancreas causal relationship information 42P1 as the corresponding causal relationship information 61. The first liver causal relationship information 42L1 and the first pancreas causal relationship information 42P1 have in common the "cholestasis" event 85. That is, the first liver causal relationship information 42L1 and the first pancreas causal relationship information 42P1 are an example of "corresponding causal relationship information in which a common event is present" and "corresponding causal relationship information for different organs" according to the technology of the present disclosure. Also, the "cholestasis" event 85 is an example of a "common event" according to the technology of the present disclosure.

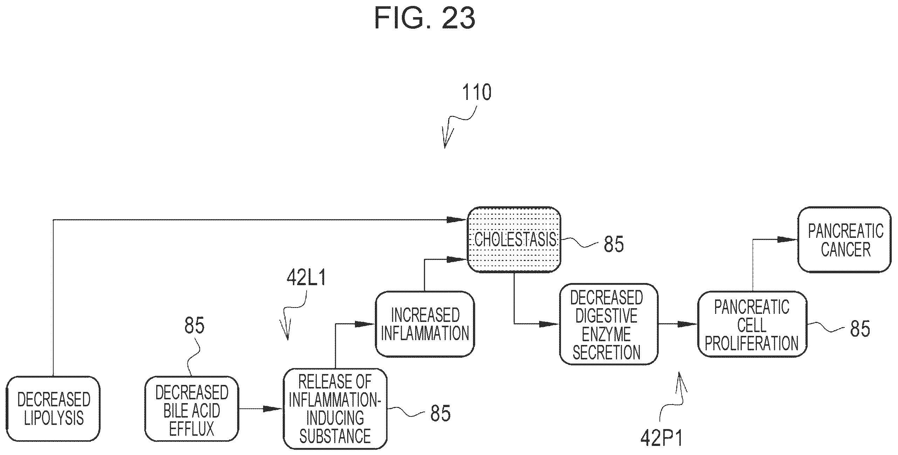

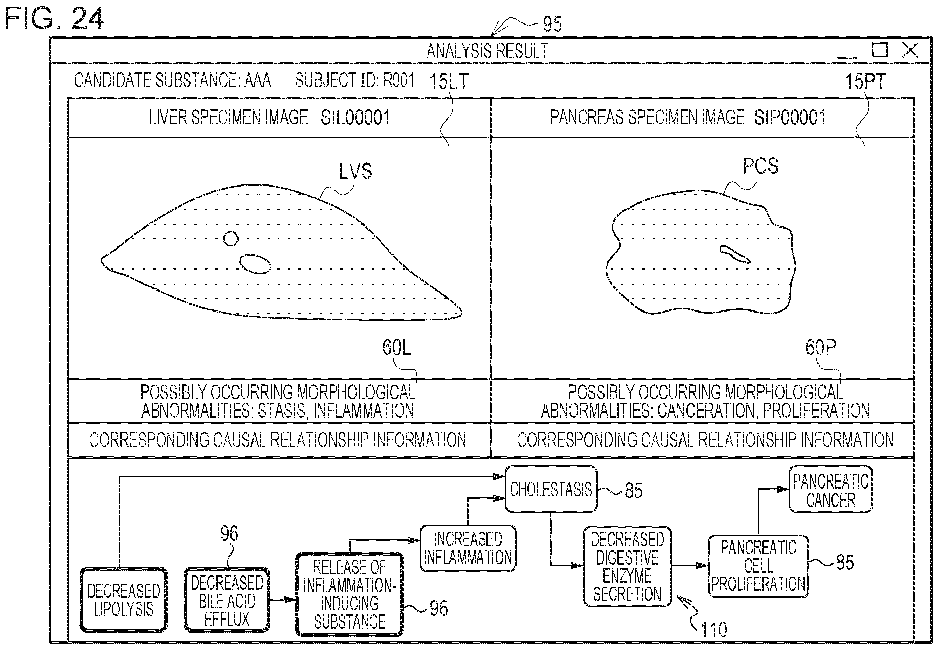

In this case, as illustrated by way of example in Fig. 23, the display control unit 53 unites the first liver causal relationship information 42L1 and the first pancreas causal relationship information 42P1 at the "cholestasis" event 85 to obtain united causal relationship information 110. Additionally, as illustrated by way of example in Fig. 24, the display control unit 53 carries out control to display the united causal relationship information 110 on the analysis result display screen 95.

In this way, in embodiment 3, in the case where the comparison and extraction unit 52 extracts multiple pieces of corresponding causal relationship information 61 and a common event 85 is present in at least two of the extracted multiple pieces of corresponding causal relationship information 61, the display control unit 53 carries out control to display the corresponding causal relationship information 61 in which the common event 85 is present, united at the common event 85. Therefore, the corresponding causal relationship information 61 in which the common event 85 is present can be summarized compactly, allowing the corresponding causal relationship information 61 in which the common event 85 is present to be displayed in an easy-to-read manner.

Also, in the example illustrated in Fig. 23, the display control unit 53 unites corresponding causal relationship information 61 for different organs, namely the first liver causal relationship information 42L1 and the first pancreas causal relationship information 42P1. Therefore, the corresponding causal relationship information 61 for different organs can be summarized compactly, allowing the corresponding causal relationship information 61 for different organs to be displayed in an easy-to-read manner.

Note that three or more pieces of corresponding causal relationship information 61 may also be united. Embodiments 2-1 and 2_2 above may also be applied to the present embodiment 3. In this case, the display control unit 53 derives a confidence level for the united causal relationship information 110.

### Embodiment 4

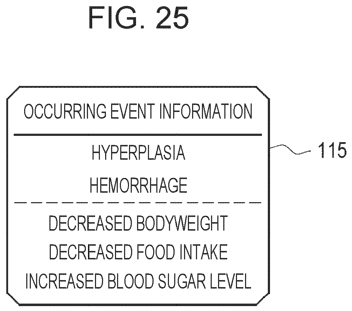

Embodiment 1 above and the like illustrate an example in which occurring events represent morphological abnormalities, but the configuration is not limited thereto. Like the occurring event information 115 illustrated by way of example in Fig. 25, occurring events may also represent changes in the bodyweight of the subject S, changes in the food intake (feed intake) by the subject S, and changes in the result of a clinical chemistry test on the subject S after administration of the candidate substance 11. Changes in the bodyweight of the subject S are "decreased bodyweight" illustrated in the drawing and "increased bodyweight". Changes in the food intake by the subject S are "decreased food intake" illustrated in the drawing and "increased food intake". Changes in the result of a clinical chemistry test on the subject S are "increased blood sugar level" illustrated in the drawing and "decreased blood sugar level". Note that changes in the result of a clinical chemistry test on the subject S may also be "increased cholesterol level" and "decreased cholesterol level", "increased uric acid level" and "decreased uric acid level", "increased gamma-glutamyl transpeptidase (γ-GTP)" and "decreased γ-GTP", or the like.

In this way, in embodiment 4, occurring events represent changes in the bodyweight of the subject S, changes in the food intake by the subject S, and changes in the result of a clinical chemistry test on the subject S. Therefore, causal relationship information 42 containing events 85 involving a change in the bodyweight of the subject S, a change in the food intake by the subject S, and a change in the result of a clinical chemistry test on the subject S can be extracted as the corresponding causal relationship information 61. If occurring events representing changes in the bodyweight of the subject S, changes in the food intake by the subject S, and changes in the result of a clinical chemistry test on the subject S are further added to occurring events representing morphological abnormalities, as in the occurring event information 115 illustrated in Fig. 25, the number of variations of the corresponding causal relationship information 61 can be increased.

Note that occurring events representing changes in the bodyweight of the subject S, changes in the food intake by the subject S, and changes in the result of a clinical chemistry test on the subject S may also be used instead of occurring events representing morphological abnormalities. Also, the occurring events may represent one or two from among changes in the bodyweight of the subject S, changes in the food intake by the subject S, and changes in the result of a clinical chemistry test on the subject S, rather than all of the above.

### Embodiment 5

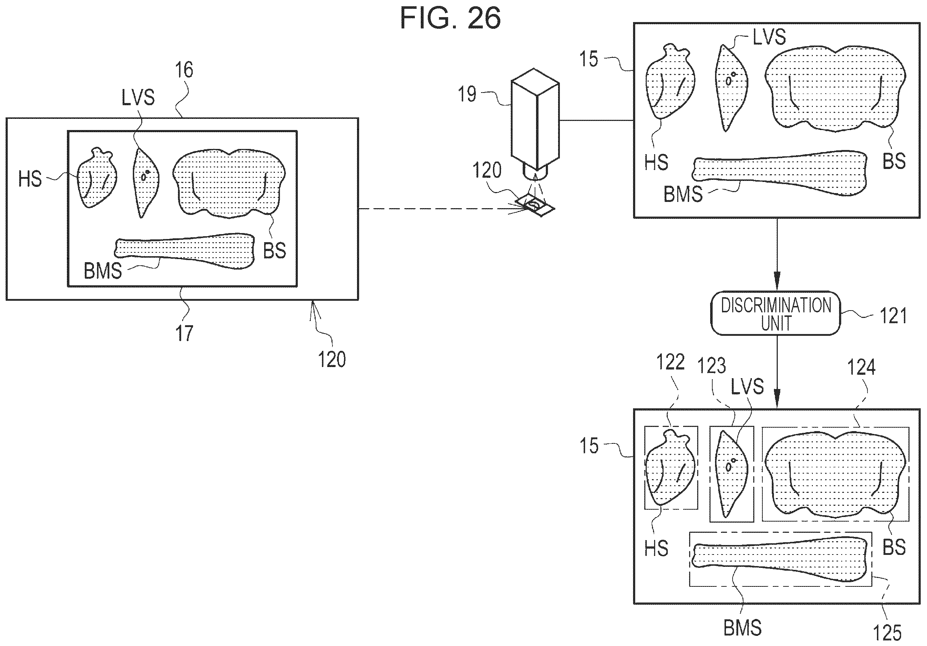

As illustrated by way of example in Fig. 26, embodiment 5 involves handling a slide specimen 120 in which tissue specimens of multiple types of organs are placed on a single microscope slide 16. Fig. 26 illustrates an example in which a heart specimen HS, a brain specimen BS, and a bone marrow specimen BMS are placed in addition to the liver specimen LVS. In this case, the specimen image 15 shows the heart specimen HS, the brain specimen BS, and the bone marrow specimen BMS in addition to the liver specimen LVS.

The CPU 32 of the information processing device 10 according to embodiment 5 functions as a discrimination unit 121 in addition to each of the processing units 50-54 in embodiment 1 above. The discrimination unit 121 discriminates the tissue specimen of each organ from the specimen image 15 by using a template or a machine learning model for discriminating tissue specimens of organs, for example. The discrimination unit 121 outputs coordinate information for frames 122-125 surrounding the tissue specimen of each organ as a discrimination result. The frame 122 is a frame surrounding the heart specimen HS, and the frame 123 is a frame surrounding the liver specimen LVS. The frame 124 is a frame surrounding the brain specimen BS, and the frame 125 is a frame surrounding the bone marrow specimen BMS.

In this way, in embodiment 5, the specimen image 15 is an image obtained by capturing the slide specimen 120 on which tissue specimens of multiple types of organs are placed. The discrimination unit 121 discriminates the tissue specimen of each organ from such a specimen image 15. This allows for compatibility with the slide specimen 120 on which tissue specimens of multiple types of organs are placed. Slide specimens 120 on which tissue specimens of multiple types of organs are placed, as in the present embodiment, are more common than slide specimens 18 on which a tissue specimen of single organ is placed, as in embodiment 1 above. This makes it possible to perform processing that is more in line with common practice.

Frames indicating the tissue specimen of each organ in the specimen image 15 may also be defined manually by the pathologist PT.

Portions where a morphological abnormality is occurring may also be indicated clearly by applying color or the like in the target specimen images 15LT and 15PT displayed on the analysis result display screen 95.

The output form of the corresponding causal relationship information 61 is not limited to the form of displaying the analysis result display screen 95 illustrated by way of example on the display 12. The corresponding causal relationship information 61 may also be in the form of printed output on a paper medium, or in the form of transmitting the corresponding causal relationship information 61 as an email attachment.

The organs are not limited to the liver LV and the like illustrated by way of example. The organs may also be the stomach, the lungs, the small intestine, the large intestine, or the like. Also, the subject S is not limited to a rat. The subject S may also be a mouse, guinea pig, gerbil, hamster, ferret, rabbit, dog, cat, monkey, or the like.

The information processing device 10 may be a personal computer set up in a pharmaceutical development facility as illustrated in Fig. 1, but may also be a server computer set up in a data center independent from a pharmaceutical development facility.

In the case of configuring the information processing device 10 as a server computer, specimen images 15 are transmitted from a personal computer set up in each pharmaceutical development facility to the server computer over a network such as the Internet. The server computer delivers various screens such as the target designation screen 90 to the personal computer in a screen data format for web delivery created using a markup language such as Extensible Markup Language (XML), for example. The personal computer reproduces a screen for display in a web browser on the basis of the screen data, and displays the reproduced screen on a display. Note that another data description language such as JavaScript^{®} Object Notation (JSON) may also be used instead of XML.

The information processing device 10 according to the technology of the present disclosure can be used extensively throughout all phases of pharmaceutical development, from the initial phase of establishing a drug design target to the final phase of clinical trials.

A variety of configurations are possible as the hardware configuration of the computer forming the information processing device 10 according to the technology of the present disclosure. For example, the information processing device 10 can be formed from multiple computers set up as discrete hardware for the purpose of improving processing power and reliability. For example, the functions of the morphological abnormality identification unit 51 and the functions of the comparison and extraction unit 52 may be handled by two computers in a distributed manner. In this case, the two computers form the information processing device 10.

In this way, the hardware configuration of the computer of the information processing device 10 can be changed, as appropriate, according to the demanded performance in terms of processing power, security, reliability, and the like. Furthermore, not only hardware but also application programs such as the operating program 40 obviously can be duplicated or distributed and stored in multiple storage locations for the purpose of ensuring security and reliability.

The various types of processors indicated below can be used as the hardware structure of the processing unit that executes various processing such as that of the RW control unit 50, the morphological abnormality identification unit 51, the comparison and extraction unit 52, the display control unit 53, the instruction accepting unit 54, and the discrimination unit 121 in the embodiments above. The various types of processors include: the CPU 32, which is a general-purpose processor that executes software (the operating program 40) to function as any of various types of processing units, as described above; a programmable logic device (PLD) whose circuit configuration is modifiable after fabrication, such as a field-programmable gate array (FPGA); and dedicated circuitry, which is a processor having a circuit configuration designed for the specific purpose of executing a specific process, such as an application-specific integrated circuit (ASIC).

A single processing unit may be configured as any one of these various types of processors, and may also be configured as a combination of two or more processors of the same or different types (for example, a combination of multiple FPGAs and/or a combination of a CPU and an FPGA). Moreover, multiple processing units may also be configured as a single processor.

A first example of configuring multiple processing units as a single processor is a mode in which a single processor is configured as a combination of software and one or more CPUs, as typified by computers such as clients and servers, such that the processor functions as the plurality of processing units. A second example of the above is a mode utilizing a processor in which the functions of an entire system, including the multiple processing units, are achieved on a single integrated circuit (IC) chip, as typified by a system on a chip (SoC). In this way, various types of processing units are configured as a hardware structure by using one or more of the various types of processors indicated above.

More specifically, circuitry combining circuit elements such as semiconductor elements can be used as the hardware structure of these various types of processors.

From the above description, the technologies described in the following appendices can be understood.

[Appendix 1] An information processing device comprising a processor configured to:
   compare a plurality of causal relationship information indicating a causal relationship of a plurality of events representing changes in a living body with an occurring event representing a change occurring in a subject which is a living body to which a pharmaceutical candidate substance was administered;
   extract corresponding causal relationship information containing the event corresponding to the occurring event from among the plurality of causal relationship information; and
   carry out control to output the corresponding causal relationship information.
[Appendix 2] The information processing device according to appendix 1, wherein the corresponding causal relationship information is information indicating a causal relationship of a plurality of events representing changes in a living body due to the toxicity of a pharmaceutical candidate substance in the past.
[Appendix 3] The information processing device according to appendix 2, wherein the occurring event represents a morphological abnormality occurring in an organ of the subject after administration of the candidate substance.
[Appendix 4] The information processing device according to appendix 3, wherein the processor is configured to identify the type of the morphological abnormality by performing image analysis on a specimen image showing a tissue specimen of an organ of the subject.
[Appendix 5] The information processing device according to appendix 4, wherein the processor is configured to identify the types of a plurality of morphological abnormalities from a single specimen image.
[Appendix 6] The information processing device according to appendix 4 or 5, wherein the processor is configured to use, in the image analysis, a machine learning model that accepts input of the specimen image and outputs in response an indication of whether the morphological abnormality is occurring.
[Appendix 7] The information processing device according to any one of appendices 1 to 6, wherein the processor is configured to:
   in a case where a plurality of pieces of the corresponding causal relationship information are extracted,
   derive a confidence level for each of the plurality of pieces of the corresponding causal relationship information; and
   carry out control to output the corresponding causal relationship information on the basis of the confidence level.
[Appendix 8] The information processing device according to appendix 7, wherein the confidence level is high to the extent that the corresponding causal relationship information contains a large number of events corresponding to the occurring event.
[Appendix 9] The information processing device according to appendix 7 or 8, wherein
   the occurring event represents a morphological abnormality occurring in an organ of the subject after administration of the candidate substance, and
   the processor is configured to:
      identify the type of the morphological abnormality by using a machine learning model that accepts input of a specimen image showing a tissue specimen of an organ of the subject and outputs in response the type of the morphological abnormality; and
      derive the confidence level on the basis of a predicted probability of whether the morphological abnormality is occurring according to the machine learning model.
[Appendix 10] The information processing device according to any one of appendices 1 to 9, wherein the processor is configured to:
   in a case where a plurality of pieces of the corresponding causal relationship information are extracted and a common event is present in at least two of the extracted plurality of pieces of corresponding causal relationship information,
   carry out control to output the corresponding causal relationship information in which the common event is present, united at the common event.
[Appendix 11] The information processing device according to appendix 10, wherein
   the causal relationship information is prepared for each of a plurality of organs, and
   the processor is configured to carry out control to output the corresponding causal relationship information in which the common event is present, the corresponding causal relationship information being for different organs, united at the common event.
[Appendix 12] The information processing device according to any one of appendices 1 to 11, wherein the occurring event represents at least one from among a change in bodyweight of the subject, a change in food intake by the subject, and a change in the result of a clinical chemistry test on the subject.
[Appendix 13] The information processing device according to any one of appendices 1 to 12, wherein
   the causal relationship information contains an event involving a molecular change in the living body, and
   the processor is configured to distinguish an event involving a molecular change in the living body from another event when carrying out control to output the corresponding causal relationship information.

The technology of the present disclosure may also be an appropriate combination of the various embodiments and/or various modifications described above. Obviously, the technology of the present disclosure is not limited to the embodiments above, and any of various configurations may be adopted within a scope that does not depart from the gist of the technology of the present disclosure. Furthermore, the technology of the present disclosure further extends to a program in addition to a storage medium storing the program in a non-transitory way.

The descriptions and illustrations given above are detailed descriptions of portions related to the technology of the present disclosure, and are nothing more than examples of the technology of the present disclosure. For example, the above descriptions pertaining to configuration, function, action, and effect are descriptions pertaining to one example of the configuration, function, action, and effect of portions related to the technology of the present disclosure. Needless to say, unnecessary portions may be deleted and new elements may be added or substituted with respect to the descriptions and illustrations given above, insofar as the result does not depart from the gist of the technology of the present disclosure. Also, to avoid confusion and to facilitate understanding of the portions related to the technology of the present disclosure, in the descriptions and illustrations given above, description is omitted in regard to common technical knowledge and the like that does not require particular explanation to enable implementation of the technology of the present disclosure.

In this specification, "A and/or B" is synonymous with "at least one of A or B". That is, "A and/or B" means that: A only is a possibility; B only is a possibility; and a combination of A and B is a possibility. Also, in this specification, the same way of thinking as for "A and/or B" also applies when three or more matters are expressively linked using "and/or".

All documents, patent applications, and technical standards mentioned in this specification are incorporated by reference herein to the same extent that individual documents, patent applications, and technical standards are specifically and individually noted as being incorporated by reference.

## Claims

1. An information processing device comprising a processor configured to:
compare a plurality of causal relationship information indicating a causal relationship of a plurality of events representing changes in a living body with an occurring event representing a change occurring in a subject which is a living body to which a pharmaceutical candidate substance was administered;
extract corresponding causal relationship information containing the event corresponding to the occurring event from among the plurality of causal relationship information; and
carry out control to output the corresponding causal relationship information.

2. The information processing device according to claim 1, wherein the corresponding causal relationship information is information indicating a causal relationship of a plurality of events representing changes in a living body due to the toxicity of a pharmaceutical candidate substance in the past.

3. The information processing device according to claim 2, wherein the occurring event represents a morphological abnormality occurring in an organ of the subject after administration of the candidate substance.

4. The information processing device according to claim 3, wherein the processor is configured to identify the type of the morphological abnormality by performing image analysis on a specimen image showing a tissue specimen of an organ of the subject.

5. The information processing device according to claim 4, wherein the processor is configured to identify the types of a plurality of morphological abnormalities from a single specimen image.

6. The information processing device according to claim 4, wherein the processor is configured to use, in the image analysis, a machine learning model that accepts input of the specimen image and outputs in response an indication of whether the morphological abnormality is occurring.

7. The information processing device according to claim 1, wherein the processor is configured to:
in a case where a plurality of pieces of the corresponding causal relationship information are extracted,
derive a confidence level for each of the plurality of pieces of the corresponding causal relationship information; and
carry out control to output the corresponding causal relationship information on the basis of the confidence level.

8. The information processing device according to claim 7, wherein the confidence level is high to the extent that the corresponding causal relationship information contains a large number of events corresponding to the occurring event.

9. The information processing device according to claim 7, wherein
the occurring event represents a morphological abnormality occurring in an organ of the subject after administration of the candidate substance, and
the processor is configured to:
identify the type of the morphological abnormality by using a machine learning model that accepts input of a specimen image showing a tissue specimen of an organ of the subject and outputs in response the type of the morphological abnormality; and
derive the confidence level on the basis of a predicted probability of whether the morphological abnormality is occurring according to the machine learning model.

10. The information processing device according to claim 1, wherein the processor is configured to:
in a case where a plurality of pieces of the corresponding causal relationship information are extracted and a common event is present in at least two of the extracted plurality of pieces of corresponding causal relationship information,
carry out control to output the corresponding causal relationship information in which the common event is present, united at the common event.

11. The information processing device according to claim 10, wherein
the causal relationship information is prepared for each of a plurality of organs, and
the processor is configured to carry out control to output the corresponding causal relationship information in which the common event is present, the corresponding causal relationship information being for different organs, united at the common event.

12. The information processing device according to claim 1, wherein the occurring event represents at least one from among a change in bodyweight of the subject, a change in food intake by the subject, and a change in the result of a clinical chemistry test on the subject.

13. The information processing device according to claim 1, wherein
the causal relationship information contains an event involving a molecular change in the living body, and
the processor is configured to distinguish an event involving a molecular change in the living body from another event when carrying out control to output the corresponding causal relationship information.

14. An operating method for an information processing device, the operating method comprising:
comparing a plurality of causal relationship information indicating a causal relationship of a plurality of events representing changes in a living body with an occurring event representing a change occurring in a subject which is a living body to which a pharmaceutical candidate substance was administered;
extracting corresponding causal relationship information containing the event corresponding to the occurring event from among the plurality of causal relationship information; and
carrying out control to output the corresponding causal relationship information.

15. An operating program for an information processing device, the operating program causing a computer to execute a process comprising:
comparing a plurality of causal relationship information indicating a causal relationship of a plurality of events representing changes in a living body with an occurring event representing a change occurring in a subject which is a living body to which a pharmaceutical candidate substance was administered;
extracting corresponding causal relationship information containing the event corresponding to the occurring event from among the plurality of causal relationship information; and
carrying out control to output the corresponding causal relationship information.
